# EUROPÄISCHE PATENTSCHRIFT

(11) **EP 0 688 757 B1**
(45) Veröffentlichungstag und Bekanntmachung des Hinweises auf die Patenterteilung: **08.03.2000**
(21) Anmeldenummer: 95109414.3
(22) Anmeldetag: 19.06.1995
(51) Int. Cl.: C07C 67/343, C07C 69/738, C07C 69/618, C07C 47/548, C07C 49/796, C07C 69/65, C07C 205/34

(54) **Verfahren zur Herstellung von aromatischen Olefinen unter Katalyse von Palladacyclen**
Process for the preparation of aromatic olefines by catalysis of palladacycles
Procédé de fabrication d'oléfines aromatiques par catalyse de paladacycles

(30) Priorität: 22.06.1994 DE 4421730
(43) Veröffentlichungstag der Anmeldung: 27.12.1995
(73) Patentinhaber: Aventis Research & Technologies GmbH & Co. KG, 65926 Frankfurt am Main (DE)
(72) Erfinder: Beller, Matthias, Dr., D-65510 Idstein (DE); Fischer, Hartmut, Dr., D-65719 Hofheim (DE); Herrmann, Wolfgang, Anton, Prof. Dr., D-85354 Freising (DE); Brossmer, Christoph, Dr., D-60318 Frankfurt (DE)

(56) Entgegenhaltungen:
- EP-A- 0 564 919
- CHEMICAL ABSTRACTS, vol. 101, no. 12, 17.September 1984 Columbus, Ohio, US; abstract no. 101670c, RHEINGOLD ARNOLD L. ET AL. 'CRYSTAL AND MOLECULAR STRUCTURE OF BIS(MU-IODO)BIS(O-(DI-O-TOLYLPHOSPHINO)BEN ZYL)DIPALLADIUM(II), A PRODUCT OF THE CYCLOPALLADATION FOR TRANS-((O-TOL)3P)2PDI2' Seite 680; Spalte LINKS; & ORGANOMETALLICS, Bd. 3, Nr. 9, 1984 Seiten 1414-1417,

## Beschreibung

Die vorliegende Erfindung betrifft ein neues Verfahren zur Herstellung von aromatischen Olefinen mit neuartigen Katalysatoren, sogenannten Palladacyclen.

Aromatische Olefine, insbesondere Zimtsäurederivate, Styrole, Stilbene haben technische Bedeutung als Feinchemikalien, Ausgangsprodukte für Polymere, UV-Absorber und Wirkstoffvorprodukte.

Eine häufig angewandte Methode zur Synthese von aromatischen Olefinen im universitären Bereich ist die Heck-Reaktion, bei der Iod- oder Bromaromaten und in Ausnahmefällen Chloraromaten mit Olefinen in Gegenwart von Palladiumkatalysatoren umgesetzt werden. Übersichten, die diese Methodik beschreiben, findet man bspw. in R.F. Heck, Acc. Chem. Res. 1979, 12, 146; R.F. Heck, Org. React. 1982, 27, 345; R.F. Heck, Palladium Reagents in Synthesis, Academic Press, London 1985.

Katalysatoren, die im Rahmen der Heck-Reaktion verwendet werden, sind Palladiumverbindungen. Obwohl sowohl Palladium(II)- als auch Palladium(O)-Komplexe bei Heck-Reaktionen eingesetzt werden, ist es doch allgemein akzeptiert, daß lediglich Palladium(O)-Verbindungen die eigentlichen Katalysatoren der Reaktion sind. Insbesondere formuliert man in der Literatur koordinativ ungesättigte 14-Elektronen Palladium(O)-Spezies, welche in der Regel mit schwachen Donorliganden wie Phosphanen stabilisiert werden.

Trotz der Vielzahl von Veröffentlichungen auf dem Gebiet der Heck-Reaktion sind bisher keine Beispiele für eine technische Umsetzung der Methodik bekannt. Dies ist darauf zurückzuführen, daß die beschriebenen Katalysatorsysteme häufig nur mit nicht ökonomischen Ausgangsmaterialien wie Iodaromaten befriedigende katalytische Wechselzahlen ("turnover numbers") ergeben. Ansonsten müssen bei Bromaromaten und insbesondere bei Chloraromaten generell große Mengen an Katalysator - allgemein 1-5 Mol-% - zugesetzt werden, um technisch nutzbare Umsätze zu erzielen. Aufgrund der Komplexität der Reaktionsgemische ist zudem kein einfaches Katalysatorrecycling möglich, so daß auch die Katalysatorkosten in der Regel einer technischen Realisierung entgegenstehen.

Daher bestand ein großer Bedarf nach einem Verfahren, das die genannten Nachteile nicht aufweist, für die technische Durchführung geeignet ist und aromatische Olefine in hoher Ausbeute und Reinheit liefert.

Diese Aufgabe wird gelöst durch ein Verfahren zur Herstellung von monofunktionellen, bi- und polyfunktionellen aromatischen Olefinen der Formel (I) 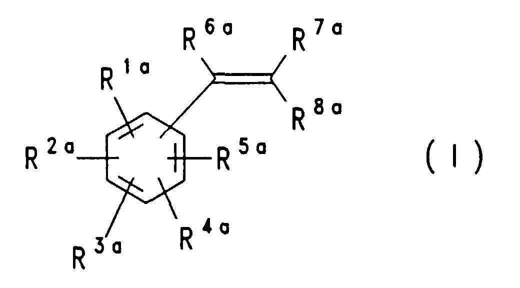 worin
R^{1a} bis R^{5a} unabhängig voneinander Wasserstoff, C₁-C₈-Alkyl, Alkoxy-(C₁-C₈), Acyloxy-(C₁-C₈), OPhenyl, Phenyl, Fluor, Chlor, Brom, Iod, OH, NO₂, OSO₂CF₃, CN, COOH, CHO, SO₃H, SO₂R, SOR, NH₂, NHAlkyl (C₁-C₈), N-Alkyl₂-(C₁-C₈), CHal₃, NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NCOO-Alkyl-(C₁-C₄), CO-Phenyl, COO-Phenyl, CHCH-CO₂Alkyl-(C₁-C₈), CHCHCO₂H, PO-Phenyl₂, PO-Alkyl₂-(C₁-C₄), C₄), wobei einer der Reste R^{1a} bis R^{5a} auch 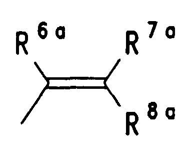 darstellen kann,
R^{6a} Wasserstoff, Alkyl(C₁-C₈), Phenyl, O-Alkyl-(C₁-C₈), Fluor
R^{7a} und R^{8a} unabhängig voneinander Wasserstoff, CN, CO₂H, CO₂-Alkyl--(C₁-C₈), CONH₂, CONH-Alkyl-(C₁-C₄), CON(Alkyl)₂-(C₁-C₄), Fluor, CO₂-Phenyl, Alkyl, (C₁-C₈)-Phenyl, PO(Phenyl), PO(Alkyl-(C₁-C₄))₂, CO-Phenyl, CO-Alkyl-(C₁-C₄), O-Alkyl-(C₁-C₄), NH-Alkyl-(C₁-C₄), PO₃H, SO₃H, SO₃-Alkyl-(C₁-C₄), SO₂-Alkyl-(C₁-C₄), O-Phenyl,
bedeuten, durch Umsetzung von Halogenaromaten der allgemeinen Formel (II) 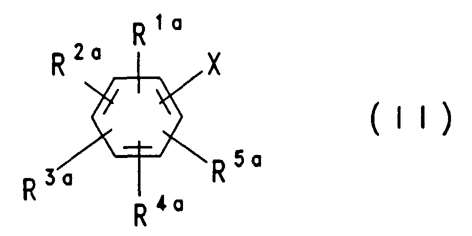 mit Olefinen der allgemeinen Formel (III) 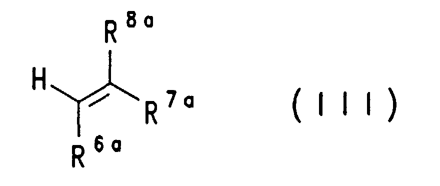 worin
R^{1a} bis R^{8a} die angegebene Bedeutung besitzen, wobei einer der Reste R^{1a} bis R^{5a} auch für X stehen kann und X lod, Brom, Chlor, OSO₂CF₃, OSO₂Phenyl, OSO₂CH₃ bedeutet, dadurch gekennzeichnet, daß man als Katalysator eine Palladiumverbindung der allgemeinen Formel (IV) 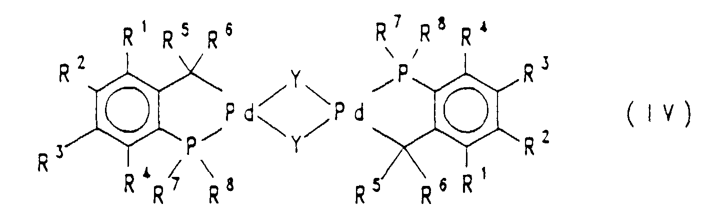 worin
R¹, R², R³, R⁴, R⁵, R⁶ unabhängig voneinander Wasserstoff, (C₁-C₄)-Alkyl, (C₅-C₈)-Cycloalkyl, (C₁-C₄)-Alkoxy, Fluor, NH₂, NH-Alkyl(C₁-C₄), N(Alkyl)₂-(C₁-C₄), CO₂Alkyl-(C₁-C₄), OCO-Alkyl-(C₁-C₄), oder Phenyl bedeuten,
oder R¹ und R², R² und R³, R³ und R⁴, R⁵ und R⁶ zusammen einen aliphatischen oder aromatischen Ring bilden, und
R⁷, R⁸ (C₁-C₈)-Alkyl, (C₃-C₁₂)-Cycloalkyl, substituiertes oder unsubstituiertes Aryl sind,
Y ein Anion einer anorganischen oder organischen Säure bedeutet, mit einem Verhältnis Pd : P = 1 : 1 einsetzt.

In vielen Fällen haben sich Verbindungen der Formel (IV), worin R¹ bis R⁶ Wasserstoff, Alkyl(C₁-C₄), Phenyl, Cycloalkyl-(C₅-C₈), R⁷ und R⁸ Phenyl, Tolyl, Xylyl, Mesityl, Alkyl(C₁-C₈) und Cycloalkyl(C₅-C₈) bedeuten und Y für Acetat, Propionat, Benzoat, Chlorid, Bromid, lodid, Fluorid, Sulfat, Hydrogensulfat, Nitrat, Phosphat, Tetrafluoroborat, Tosylat, Mesylat, Acetylacetonat, Hexafluoracetylacetonat oder Pyrazolyl steht, bewährt.

Gut geeignet sind z.B. Verbindungen wo R¹ - R⁶ für H, Alkyl, Phenyl und R⁷, R⁸ für Alkyl, Phenyl, Tolyl, Mesityl und Xylyl stehen.

Sehr gute Ergebnisse liefern die Verbindungen:
trans-Di-µ-acetato-bis[o-(di-o-tolylphosphino)benzyl]dipalladium(II)
trans-Di-µ-chloro-bis[o-(di-o-tolylphosphino)benzyl]dipalladium(II)
trans-Di-µ-bromo-bis[o-(di-o-tolylphosphino)benzyl]dipalladium(II)
trans-Di-µ-iodo-bis[o-(di-o-tolylphosphino)benzyl]dipalladium(II)
trans-Di-µ-acetato-bis[o-(dimesityiphosphinol-3,5-dimethylbenzyl]dipalladium(II)
trans-Di-µ-chloro-bis[o-(dimesitylphosphino)-3,5-dimethylbenzyl]dipalladium(II)
trans-Di-µ-bromo-bis[o-(dimesitylphosphino)-3,5-dimethylbenzyl]dipalladium(II)
trans-Di-µ-iodo-bis[o-(dimesitylphosphino)-3,5-dimethylbenzyl]dipalladium(II)
trans-Di-µ-acetato-bis[o-(t-butyl-o-tolylphosphino)benzyl]dipalladium(II)
trans-Di-µ-acetato-bis[o-(di-t-butylphosphino)benzyl]dipalladium(II)
trans-Di-µ-acetato-bis[o-(cyclohexyl-o-tolylphosphino)benzyl]dipalladium(II)

Das Verfahren hat sich insbesondere für die Herstellung von Verbindungen der Formel (I) bewährt, worin bedeuten:
R^{1a} bis R^{5a} unabhängig voneinander Wasserstoff, (C₁-C₈)-Alkyl, (C₁-C₈)-Alkoxy, (C₁-C₈)-Acyloxy, Phenyl, Fluor, Chlor, NO₂, CN, COOH, CHO, SO₂R,
NH-(C₁-C₈)-Alkyl, N[(C₁-C₈)Alkyl]₂, COO-(C₁-C₈)-Alkyl, CONH₂, CO-(C₁-C₈)-Alkyl, CO-Phenyl, COO-Phenyl, PO-(Phenyl)₂, R^{6a} Wasserstoff, (C₁-C₈)-Alkyl,
R^{7a}, R^{8a} unabhängig voneinander Wasserstoff, CN, CO₂H, CO₂-(C₁-C₈)-Alkyl, CO₂-Phenyl, (C₁-C₈)-Alkyl, CO-Phenyl, CO-(C₁-C₄)-Alkyl.

Wichtig ist das Verfahren z.B. zur Herstellung von Verbindungen der Formel (I) worin bedeuten:
R^{1a} bis R^{5a} unabhängig voneinander Wasserstoff, (C₁-C₈)-Alkyl, (C₁-C₈)-Alkoxy, Phenyl, Fluor, Chlor, NO₂, CN, COOH, CHO, COO-(C₁-C₈)-Alkyl, CONH₂, CO-(C₁-C₈)-Alkyl, CO-Phenyl, PO-(Phenyl)₂, R^{6a} Wasserstoff,
R^{7a}, R^{8a} unabhängig voneinander CN, CO₂H, CO₂-(C₁-C₈)-Alkyl, CO₂-Phenyl, CO-Phenyl, CO-(C₁-C₄)-Alkyl.

Als Lösungsmittel finden im allgemeinen inerte organische Lösungsmittel Verwendung. Gut geeignet sind dipolar aprotische Lösungsmittel wie Dialkylsulfoxide, N,N-Dialkylamide von aliphatischen Carbonsäuren oder alkylierte Lactame. Hierbei sind Dimethylsulfoxid, Dimethylacetamid, Dimethylformamid und N-Methylpyrrolidon bevorzugt.

Die Reaktion läuft bei Temperaturen von 20 bis 200°C ab, in vielen Fällen hat es sich bewährt, bei Temperaturen von 60 bis 180°C, bevorzugt 100 bis 150°C zu arbeiten.

Da bei der Reaktion HX abgespalten wird, ist es vorteilhaft, diese Säure durch Zusatz einer Base abzufangen. Dazu geeignet sind primäre, sekundäre oder tertiäre Amine wie Alkylamine, Dialkylamine, Trialkylamine, die alicyclisch oder offenkettig sein können und Alkali- oder Erdalkalisalze von aliphatischen oder aromatischen Carbonsäuren oder der Kohlensäure, wie Lithium, Natrium-, Kalium-, Calcium-, Magnesiumacetat und entsprechende Carbonate oder Hydrogencarbonate.

Die eingesetzten Palladiumkatalysatoren werden in der Regel vor der eigentlichen Reaktion isoliert synthetisiert, sie können jedoch auch in situ erzeugt werden, ohne daß dadurch die anfängliche katalytische Aktivität gemindert wird. Bei längerer Reaktionführung erweisen sich die in situ Mischungen (molares Verhältnis Pd:P = 1:1) jedoch als wenig stabil und führen häufig zur Palladiumabscheidung.

Die Synthese der eingesetzten Palladiumkatalysatoren erfolgt gemäß dem Verfahren der am gleichen Tag eingereichten deutschen Patentanmeldung P 44 21 753.6.

Die eingesetzten oder sich bildenden Palladacyclen haben in der Regel einen dimeren Aufbau. Bei bestimmten Verbindungen (z.B. Y = Acetylaceton, Hexafluoracetylaceton) können jedoch auch monomere, oligomere oder gar polymere Strukturen vorliegen.

Während des Katalysezyklus wird durch Brückenspaltungsreaktionen mit anorganischen und organischen Nucleophilen die dimere Struktur aufgebrochen, so daß als eigentlich katalytisch aktive Spezies die einkernigen Komplexe der Formel (V) bzw. (VI) 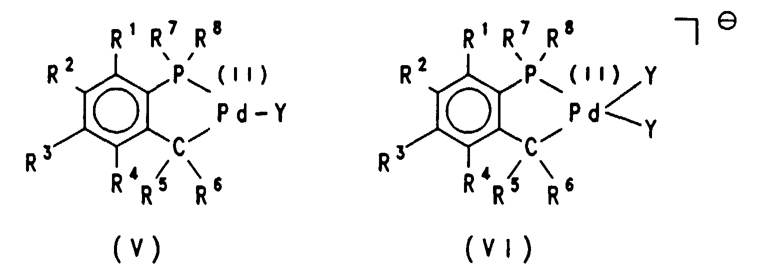 in Betracht zu ziehen sind. Die Komplexe der Formel (V) und (VI) stehen mit den tatsächlichen eingesetzten Dimeren im Austauschgleichgewicht und haben neutralen oder anionischen Charakter. Der einkernige Komplex der Formel (V) kann dabei gegebenenfalls weitere Donorliganden am Palladiumatom enthalten.

Der sehr vorteilhafte Verlauf der erfindungsgemäßen Reaktion war besonders überraschend, da nach dem Stand der Technik Palladiumkatalysatoren der Formel (IV) für die Durchführung der Heck-Reaktion als ungeeignet galten.

So stellt R.F. Heck ausdrücklich fest, daß Palladacyclen keine katalytische Aktivität für die Arylierung von Olefinen besitzen (T. Mitsudo, W. Fischetti, R.F. Heck, J. Org. Chem., Jg. 1984, Bd. 49, 1640).

Auch A.L. Rheingold und W.C. Fultz (Organometallics Jg. 1984, Bd. 3, 1414) beschreiben, daß sich bei der Heck-Reaktion von Iodaromaten mit Dienen in Gegenwart von Palladiumacetat und Tris-(o-tolyl)phosphan Palladacyclen bilden, die keine katalytische Aktivität aufweisen.

Vor diesem Hintergrund sind die Vorteile der bei den erfindungsgemäßen Verfahren eingesetzten Katalysatoren höchst unerwartet und besonders überraschend.

Die als neue Katalysatorsysteme eingesetzten Palladacyclen zeichnen sich durch sehr große Aktivität und unerwarteterweise damit einhergehende hohe Stabilität aus.

Die Stabilität der Palladacyclen in Lösung läßt sich durch Zusatz von Alkali-, Erdalkali- und Übergangsmetallsalzen der 6. bis 8. Nebengruppe erhöhen. Insbesondere der Zusatz von Halogeniden und Pseudohalogeniden (z.B. CN⁻) bewirken bei der Umsetzung von Chloraromaten signifikante Ausbeutesteigerungen (1 bis 100 %) und Standzeitverbesserungen des Homogenkatalysators. Geeignet sind auch Tri- und Tetraalkylammonium-Salze sowie entsprechende Phosphonium- und Arsonium-Salze.

So können Turnover-Werte in der Größenordnung von 100.000 und mehr realisiert werden.

Aufgrund der Katalysatoraktivitäten und -stabilität ist es somit bei bestimmten Verfahren möglich, extrem kleine Mengen an Katalysator zu verwenden, so daß die Katalysatorkosten im Vergleich zu herkömmlichen Heck-Reaktionen für den entsprechenden Prozeß nicht kostenlimitierend sind.

Außerdem bedingt der Einsatz minimalster Mengen an Katalysator ökologische Vorteile, da Abfallprodukte oder abfallproduktträchtige Aufarbeitungsverfahren vermieden werden.

Die nachstehenden Beispiele dienen zur Erläuterung des erfindungsgemäßen Verfahrens, ohne es darauf zu beschränken.

### Beispiel 1: Synthese des Katalysators

### 1. trans-Di-(µ-acetato-bis[o-(di-o-tolylphosphino)benzyl]dipalladium(II)(1)

4,5 g (20 mmol) Pd(OAc)₂ werden in 500 ml Toluol mit rotbrauner Farbe gelöst. Die Lösung wird mit 8,0 g (26,3 mmol) Tri-(o-tolyl)phosphan versetzt. Die sich rasch nach hellorange aufklarende Lösung wird 3 Minuten lang auf knapp 50°C erhitzt und dann auf Raumtemperatur abgekühlt. Das Lösungsmittel wird im Vakuum bis auf 1/4 des Volumens entfernt. Nach Zugabe von 500 ml Hexan wird der ausgefallene Niederschlag abfiltriert. Man erhält 8,8 g (93 % d. Th.) bezogen auf Pd(OAc)₂) (1) als gelben Feststoff (Schmp. > 200°C). Durch Umkristallisation aus Toluol/Hexan oder Methylenchlorid/Hexan und Filtration der Lösungen über Celite® kann (1) in Form gelber Kristallnadeln analysenrein gewonnen werden.
Elementaranalyse:
Gef.: C, 58,89 %; H, 5,06 %; P, 6,92 %; O, 6,47 %; Pd, 21,84 %;
C₄₆H₄₆O₄P₂Pd₂ (937,62)
ber.: C, 58,93 %; H, 4,94 %; P, 6,61 %; O, 6,83 %; Pd, 22,70 %;
IR (cm⁻¹, KBr): 3052m, 3007m, 2954w, 2925m *v*(CH); 1578vs *v*(µ₂-C=O), 1468s; 1630 *v*(C=C); 1578, 1416 *v*/µ₂-CO); 1341;
¹H-NMR (400 MHz, -70°C, CD₂Cl₂): δ = 7.31 (4H, m, H_{Tolyl}); 7.21 (2H, m, H_{Tolyl}); 7.12 (6H, m, H_{Tolyl}); 7.06 (2H, t, H_{Benzyl}, ³J(HH) = 7.3 Hz); 6.92 (4H, m, H_{Tolyl}); 6.70 (2H, t, H_{Benzyl}, ³J(HH) = 7.3 Hz); 6.56 (2H, t, H_{Benzyl}, ³J(HH) = 9 Hz); 6.35 (2H, dd, H_{Benzyl}, ³J(HH) = 7.9 Hz, ⁴J(PH) = 12.2 Hz); 3.00 (6H, s, CH₃); 2.81 (2H, dd, CHₐH_{b}, ²J(HₐH_{b}) = 14.0 Hz, ³J(PH) = 4.3 Hz); 2.40 (2H, dd, CHₐH_{b}, ²J(HₐH_{b}) = 14.0 Hz, ³J(PH) = 1.8 Hz); 2.10 (6H, s, CH₃); 1.91 (s, 6H, CH₃);
¹³C{¹H}-NMR (100.5 MHz, -70°C, CD₂Cl₂): δ = 178.5 (s, CH₃CO₂); 157.1 (d, C_{Ar}, J(PC) = 31.3 Hz); 141.1 (d, C_{Ar}, J(PC) = 16.0 Hz); 141,0 (d, C_{Ar}, J(PC) = 21.0 Hz); 133.0 (s, C_{Ar}); 132.5 (d, C_{Ar}, J(PC) = 4.6 Hz); 132.4 (d, C_{Ar}, J(PC) = 6.1 Hz); 131.7 (d, C_{Ar}, J(PC) = 8.8 Hz); 131.4 (d, C_{Ar}; J(PC) = 13.7); 131.3 (d, C_{Ar}, J(PC) = 9.9 Hz); 130.4 (d, C_{Ar}, J(PC) = 16.0 Hz); 129.9 (s, C_{Ar}); 129.1 (d, C_{Ar}, J(PC) = 46.2 Hz); 128.7 (s, C_{Ar}); 128.1 (d, C_{Ar}, J(PC) = 33.2 Hz); 127.6 (d, C_{Ar}, J(PC) = 23.7 HZ); 125.6 (d, C_{Ar}, J(PC) = 7.3 Hz); 125.2 (d, C_{Ar}, J(PC) = 7.3 Hz); 124.9 (d. C_{Ar}, J(PC) = 11.4 Hz); 30.8 (s, CH₂); 24.7 (d, CH₃CO₂, 4J(PC) = 3.1 Hz);23.0 (d, CH₃, 3J(PC) = 13.7 Hz); 22.2 (d, CH₃, 3J(PC) = 6.9 Hz);
³¹P{¹H}-NMR (161.9 MHz, -70°C, CD₂Cl₂): δ = 34.2 (s);
CI-MS (150 eV): m/e = 939 [M⁺ + H], 880 [M⁺-OAc], 819 [M⁺-2OAc], 714 [Pd{o-CH₂C₆H₄P(o-Tol)₂}₂⁺].

### Beispiel 2

100 mmol 4-Brombenzaldehyd, 170 mmol n-Butylacrylat, 100 mmol Natriumacetat werden in 100 ml Dimethylacetamid mit 0,005 mmol trans-Di-(µ-acetato-bis-o-(di-o-tolylphosphino)benzyl)dipalladium(II) als Katalysator 3 Stunden bei 130°C gerührt.
Ausbeute: 100 % 4-Formylzimtsäure-n-butylester

### Beispiel 3

100 mmol 4-Bromacetophenon, 150 mmol 2-Ethylhexylacrylat, 110 mmol Natriumacetat werden in 100 mmol Dimethylacetamid mit 0,005 mmol trans-Di-µ-acetato-bis-(o-(di-o-tolylphosphino)benzyl)dipalladium(II) als Katalysator 3 Stunden bei 130°C gerührt.
Ausbeute: 100 % E-4-Acetylzimtsäure-2-ethylhexylester

### Beispiel 4

100 mmol 4-Chloracetophenon, 170 mmol 2-Ethylhexylacrylat, 110 mmol Natriumacetat, 10 mmol Lithiumbromid werden in 100 ml Dimethylacetamid mit 0,05 mmol trans-Di-µ-acetato-bis-(o-(di-o-tolylphosphino)benzyl)dipalladium(II) als Katalysator 18 Stunden bei 130°C gerührt.
Ausbeute: 82 % E-4-Acetylzimtsäure-2-ethylhexylester

### Beispiel 5

100 mmol 2-Bromtoluol, 170 mmol n-Butylacrylat, 110 mmol Natriumacetat werden in 100 ml Dimethylacetamid mit 1 mmol trans-Di-µ-acetato-bis-(o-(di-o-tolylphosphino)benzyl)dipalladium (II) als Katalysator 48 Stunden bei 140°C gerührt.
Ausbeute: 92 % E-2-Methylzimtsäurebutylester

### Beispiel 6

100 mmol Brombenzol, 170 mmol Butylacrylat, 110 mmol Natriumacetat werden in 100 ml Dimethylacetamid mit 1 mmol trans-Di-µ-acetato-bis-(o-(di-o-tolylphosphino)benzyl)dipalladium (II) als Katalysator 48 Stunden bei 140°C gerührt.
Ausbeute: 96 % Zimtsäure-n-butylester

### Beispiel 7

100 mmol 4-Bromacetophenon, 150 mmol 2-Ethylhexylacrylat, 110 mmol Natriumacetat werden in 100 ml Dimethylacetamid mit 0,005 mmol trans-Di-acetato-bis-(o-(di-phenylphosphino)-4-methylbenzyl)dipalladium (II) als Katalysator 3 Stunden bei 130°C gerührt.
Ausbeute: 100 % 4-Acetylzimtsäure-2-ethylhexylester

### Beispiel 8

1.00 mmol 4-lodbrombenzol 3,0 mmol n-Butylacrylat, 2,2 mmol Natriumacetat wurden in 10 ml Dimethylacetamid mit 0,005 mmol Di-µ-acetato-bis(di-o-tolylphosphino)benzyl)dipalladium(II) als Katalysator 48 Stunden bei 140°C gerührt.
Ausbeute: 85 % (E,E')-1,4-Bis(2-butoxycarbonylvinyl)benzol

### Beispiel 9

100 mmol 4-Brombenzaldehyd, 150 mmol Acrylsäurebutylester, 110 mmol Natriumacetat werden in 80 ml Dimethylacetamid mit 0,001 mmol trans-Di-µ-acetato-bis-[o-(dimesitylphosphino)-3,5-dimethylbenzyl]-dipalladium (II)
6 Stunden bei 135°C gerührt.
Ausbeute: 99 % 4-Formylzimtsäurebutylester

### Beispiel 10

100 mmol 4-Brombenzaldehyd, 150 mmol Acrylsäurebutylester, 110 mmol Natriumacetat werden in 80 ml Dimethylacetamid mit 0,001 mmol trans-Di-µ-acetato-bis-[o-(t-butyl-o-tolylphosphino)-benzyl]-dipalladium (II)
6 Stunden bei 135°C gerührt.
Ausbeute: 100 % 4-Formylzimtsäurebutylester

### Beispiel 11

100 mmol 4-Brombenzaldehyd, 150 mmol Acrylsäurebutylester, 110 mmol Natriumacetat werden in 100 ml Dimethylacetamid mit 0,001 mmol trans-Di-µ-acetato-bis-[o-(di-t-butylphosphino)-benzyl]-dipalladium (II)
6 Stunden bei 130°C gerührt.
Ausbeute: 100 % 4-Formylzimtsäurebutylester

### Beispiel 12

100 mmol 4-Brombenzaldehyd, 150 mmol Acrylsäurebutylester, 110 mmol Natriumacetat werden in 80 ml Dimethylacetamid mit 0,001 mmol trans-Di-µ-acetato-bis-[o-(cyclohexyl-o-tolylphosphino)-benzyl]-dipalladium (II) 4 Stunden bei 135°C gerührt.
Ausbeute: 100 % 4-Formylzimtsäurebutylester

### Beispiel 13

100 mmol 4-Brombenzaldehyd, 150 mmol Acrylsäurebutylester, 110 mmol Natriumacetat werden in 80 ml Dimethylacetamid mit 0,001 mmol trans-Di-µ-bromo-bis-[o-dimesitylphosphino)-3,5-dimethylbenzyl]-dipalladium (II) 4 Stunden bei 135°C gerührt.
Ausbeute: 98 % 4-Formylzimtsäurebutylester

### Beispiel 14

100 mmol 4-Brombenzaldehyd, 150 mmol Acrylsäurebutylester, 110 mmol Natriumacetat werden in 80 ml Dimethylacetamid mit 0,001 mmol trans-Di-µ-bromo-bis-[o-(di-o-tolylphosphino)benzyl]-dipalladium(II) 6 Stunden bei 140°C gerührt.
Ausbeute: 100 % 4-Formylzimtsäurebutylester

### Beispiel 15

100 mmol 4-Brombenzaldehyd, 180 mmol Styrol, 110 mmol Natriumacetat werden in 80 ml Dimethylacetamid mit 0,1 mmol trans-Di-µ-bromo-bis-[o-(di-o-tolylphosphino)benzyl]-dipalladium(II) 6 Stunden bei 140°C gerührt.
Ausbeute: 96 % 4-Formylstilben

### Beispiel 16

100 mmol 4-Bromanisol, 150 mmol Acrylsäurebutylester, 110 mmol Natriumacetat werden in 80 ml Dimethylacetamid mit 0,1 mmol trans-Di-µ-acetato-bis[o-(cyclohexyl-o-tolylphosphino)-benzyl]dipalladium (II) 4 Stunden bei 145°C gerührt.
Ausbeute: 94 % 4-Methoxyzimtsäurebutylester

### Beispiel 17

100 mmol 4-Bromanisol, 150 mmol Acrylsäurebutylester, 110 mmol Natriumacetat werden in 80 ml Dimethylacetamid mit 0,1 mmol trans-Di-µ-iodo-bis[o-(cyclohexyl-o-tolylphosphino)-benzyl]dipalladium(II) 4 Stunden bei 145°C gerührt.
Ausbeute: 87 % 4-Methoxyzimtsäurebutylester

### Beispiel 18

25 mmol Bromacetophenon, 37 mmol Styrol, 30 mmol Natriumacetat, 0,60 g Bis(di-o-tolylphosphinobenzyl)palladiumacetat und 2 mg Di-tert.butylphenol werden in 50 ml Dimethylacetamid bei 130°C bis zum vollständigen Umsatz gerührt. Man erhält nach Abfiltrieren der Salze, Fällung des Rohproduktes mit Wasser und Umkristallisation aus Aceton/Wasser 87 % Produkt.
Ausbeute: 87 % 4-Acetylstilben

### Beispiel 19

310 mmol 1-Brom-2,4-difluorbenzol, 465 mmol Acrylsäurebutylester, 372 mmol Natriumacetat werden in 150 ml Dimethylacetamid mit 0,31 mmol Bis-(di-o-tolylphosphinobenzyl)palladiumacetat 16 Stunden bei 130°C gerührt. Ausbeute: 82 % 2,4-Difluorzimtsäurebutylester

### Beispiel 20

20 mmol 4-Bromnitrobenzol, 40 mmol Butylvinylether, 30 mmol Triethylamin werden in 20 ml Xylol mit 0,2 mmol Bis(di-o-tolylphosphinobenzyl)palladiumacetat 16 Stunden bei 140°C gerührt.
Umsatz: 100 %
Selektivität: 9:1 = 2-(cis,trans)-Butoxy-1-(4-nitrophenyl)ethylen/1-Butoxy-1-(4-nitrophenyl)-ethylen

## Patentansprüche

1. Ein Verfahren zur Herstellung von monofunktionellen, bi- und polyfunktionellen aromatischen Olefinen der Formel (I) 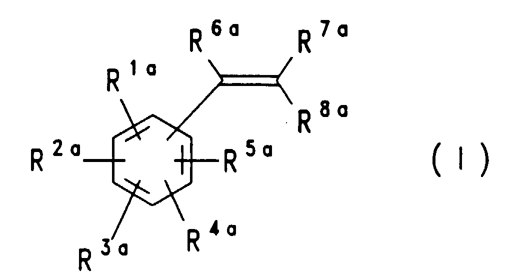 worin
R^{1a} bis R^{5a} unabhängig voneinander Wasserstoff, C₁-C₈-Alkyl, Alkoxy-(C₁-C₈), Acyloxy-(C₁-C₈), OPhenyl, Phenyl, Fluor, Chlor, Brom, lod, OH, NO₂, OSO₂CF₃, CN, COOH, CHO, SO₃H, SO₂R, SOR, NH₂, NHAlkyl (C₁-C₈), N-Alkyl₂-(C₁-C₈), CHal₃, NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NCOO-Alkyl-(C₁-C₄), CO-Phenyl, COO-Phenyl, CHCH-CO₂Alkyl-(C₁-C₈), CHCHCO₂H, PO-Phenyl₂, PO-Alkyl₂-(C₁-C₄), wobei einer der Reste R^{1a} bis R^{5a} auch 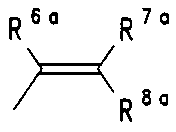 darstellen kann,
R^{6a} Wasserstoff, Alkyl(C₁-C₈), Phenyl, O-Alkyl-(C₁-C₈), Fluor
R^{7a} und R^{8a} unabhängig voneinander Wasserstoff, CN, CO₂H, CO₂-Alkyl--(C₁-C₈), CONH₂, CONH-Alkyl-(C₁-C₄), CON(Alkyl)₂-(C₁-C₄), Fluor, CO₂-Phenyl, Alkyl, (C₁-C₈)-Phenyl, PO(Phenyl), PO(Alkyl-(C₁-C₄))₂, CO-Phenyl, CO-Alkyl-(C₁-C₄), O-Alkyl-(C₁-C₄), NH-Alkyl-(C₁-C₄), PO₃H, SO₃H, SO₃-Alkyl-(C₁-C₄), SO₂-Alkyl-(C₁-C₄), O-Phenyl,
bedeuten, durch Umsetzung von Halogenaromaten der allgemeinen Formel (II) 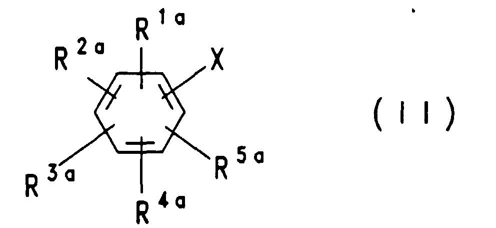 mit Olefinen der allgemeinen Formel (III) 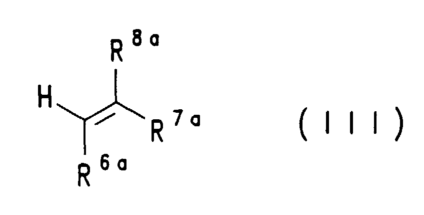 worin
R^{1a} bis R^{8a} die angegebene Bedeutung besitzen, wobei einer der Reste R^{1a} bis R^{5a} auch für X stehen kann und X lod, Brom, Chlor, OSO₂CF₃, OSO₂Phenyl, OSO₂CH₃ bedeutet, dadurch gekennzeichnet, daß man als Katalysator eine Palladiumverbindung der allgemeinen Formel (IV) 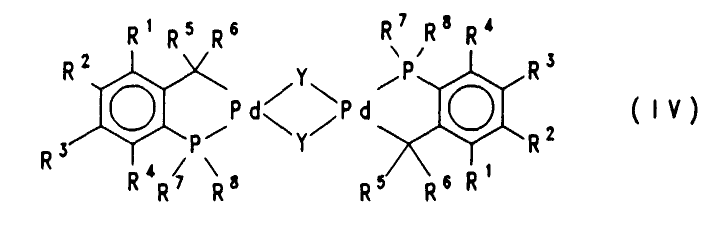 worin
R¹, R², R³, R⁴, R⁵, R⁶ unabhängig voneinander Wasserstoff, (C₁-C₄)-Alkyl, (C₅-C₈)-Cycloalkyl, (C₁-C₄)-Alkoxy, Fluor, NH₂, NH-Alkyl(C₁-C₄), N(Alkyl)₂-(C₁-C₄), CO₂Alkyl-(C₁-C₄), OCO-Alkyl-(C₁-C₄), oder Phenyl bedeuten,
oder R¹ und R², R² und R³, R³ und R⁴, R⁵ und R⁶ zusammen einen aliphatischen oder aromatischen Ring bilden, und
R⁷, R⁸ (C₁-C₈)-Alkyl, (C₃-C₁₂)-Cycloalkyl, substituiertes oder unsubstituiertes Aryl sind
Y ein Anion einer anorganischen oder organischen Säure bedeutet, mit einem Verhältnis Pd : P = 1 : 1 einsetzt.

2. Verfahren nach Anspruch 1, dadurch gekennzeichnet, daß in Formel (IV) R¹ bis R⁶ unabhängig voneinander Wasserstoff, (C₁-C₄)-Alkyl, (C₅-C₈)-Cycloalkyl,
R⁷, R⁸ Phenyl, Tolyl, Xylyl, Mesityl, Alkyl-(C₁-C₈), Cycloalkyl-(C₅-C₈) bedeuten und
Y für Acetat, Propionat, Benzoat, Chlorid, Bromid, lodid, Fluorid, Sulfat, Hydrogensulfat, Nitrat, Phosphat, Tetrafluoroborat, Tosylat, Mesylat, Acetylacetonat, Hexafluoracetylacetonat oder Pyrazolyl steht.

3. Verfahren nach Anspruch 1, dadurch gekennzeichnet, daß als Katalysator die Verbindungen
trans-Di-µ-acetato-bis[o-(di-o-tolylphosphino)benzyl]dipalladium(II)
trans-Di-µ-chloro-bis[o-(di-o-tolylphosphino)benzyl]dipalladium(II)
trans-Di-µ-bromo-bis[o-(di-o-tolylphosphino)benzyl]dipalladium(II)
trans-Di-µ-iodo-bis[o-(di-o-tolylphosphinolbenzyl]dipalladium(II)
trans-Di-µ-acetato-bis[o-(dimesitylphosphino)-3,5-dimethylbenzyl]dipalladium(II)
trans-Di-µ-chloro-bis[o-(dimesitylphosphino)-3,5-dimethylbenzyl]dipalladium(II)
trans-Di-µ-bromo-bis[o-(dimesitylphosphino)-3,5-dimethylbenzyl]dipalladium(II)
trans-Di-µ-iodo-bis[o-(dimesitylphosphino)-3,5-dimethylbenzyl]dipalladium(II)
trans-Di-µ-acetato-bis[o-(t-butyl-o-tolylphosphino)benzyl]dipalladium(II)
trans-Di-µ-acetato-bis[o-(di-t-butylphosphino)benzyl]dipalladium(II)
trans-Di-µ-acetato-bis[o-(cyclohexyl-o-tolylphosphino)benzyl]dipalladium(II) eingesetzt werden.

4. Verfahren nach mindestens einem der Ansprüche 1 bis 3, dadurch gekennzeichnet, daß der Katalysator in situ hergestellt wird.

5. Verfahren nach mindestens einem der Ansprüche 1 bis 4, dadurch gekennzeichnet, daß in Formel (I) bedeuten:
R^{1a} bis R^{5a} unabhängig voneinander Wasserstoff, (C₁-C₈)-Alkyl, (C₁-C₈)-Alkoxy, (C₁-C₈)-Acyloxy, Phenyl, Fluor, Chlor, NO₂, CN, COOH, CHO, SO₂R, NH-(C₁-C₈)-Alkyl, N[(C₁-C₈)Alkyl]₂, COO-(C₁-C₈)-Alkyl, CONH₂, CO-(C₁-C₈)-Alkyl, CO-Phenyl, COO-Phenyl, PO-(Phenyl)₂,
R^{6a} Wasserstoff, (C₁-C₈)-Alkyl,
R^{7a}, R^{8a} unabhängig voneinander Wasserstoff, CN, CO₂H, CO₂-(C₁-C₈)-Alkyl, CO₂-Phenyl, (C₁-C₈)-Alkyl, CO-Phenyl, CO-(C₁-C₄)-Alkyl.

6. Verfahren nach mindestens einem der Ansprüche 1 bis 4, dadurch gekennzeichnet, daß in Formel (I) bedeuten:
R^{1a} bis R^{5a} unabhängig voneinander Wasserstoff, (C₁-C₈)-Alkyl, (C₁-C₈)-Alkoxy, Phenyl, Fluor, Chlor, NO₂, CN, COOH, CHO, COO-(C₁-C₈)-Alkyl, CONH₂, CO-(C₁-C₈)-Alkyl, CO-Phenyl, PO-(Phenyl)₂,
R^{6a} Wasserstoff,
R^{7a}, R^{8a} unabhängig voneinander CN, CO₂H, CO₂-(C₁-C₈)-Alkyl, CO₂-Phenyl, CO-Phenyl, CO-(C₁-C₄)-Alkyl.

7. Verfahren nach mindestens einem der Ansprüche 1 bis 6, dadurch gekennzeichnet, daß als Lösungsmittel dipolar aprotische Lösungsmittel, insbesondere Dialkylsulfoxide, N,N-Dialkylamide, alkylierte Lactame, bevorzugt Diemthylsulfoxid, Dimethylacetamid, Dimethylformamid oder N-Methylpyrrolidon eingesetzt werden.

8. Verfahren nach mindestens einem der Ansprüche 1 bis 7, dadurch gekennzeichnet, daß die Reaktion bei Temperaturen von 20 bis 200°C, insbesondere 60 bis 180°C, bevorzugt 100 bis 150°C durchgeführt wird.

9. Verfahren nach mindestens einem der Ansprüche 1 bis 8, dadurch gekennzeichnet, daß die bei der Reaktion entstehende Säure HX durch Zusatz einer Base, insbesondere eines Amins oder eines Alkali oder Erdalkalimetallsalzes einer schwachen Säure abgefangen wird.

10. Verfahren nach Anspruch 9, dadurch gekennzeichnet, daß als Base Alkylamine oder die Carbonate, Hydrogencarbonate oder Acetate von Lithium, Natrium, Kalium, Calcium oder Magnesium eingesetzt werden.

11. Verfahren nach mindestens einem der Ansprüche 1 bis 10, dadurch gekennzeichnet, daß Salze von Halogeniden und Pseudohalogeniden der Alkali-, Erdalkalimetalle und Metalle der 6. bis 8. Nebengruppe zugesetzt werden.

12. Verfahren nach mindestens einem der Ansprüche 1 bis 10, dadurch gekennzeichnet, daß Tri- oder Tetraalkylammonium, -phosphonium oder - arsoniumsalze zugesetzt werden.

## Claims

1. A process for preparing monofunctional, bifunctional and polyfunctional aromatic olefins of the formula (I) 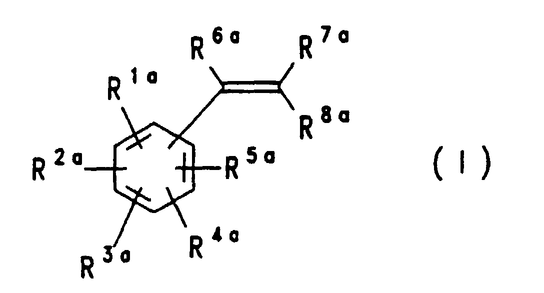 where
R^{1a} to R^{5a} are, independently of one another, hydrogen, C₁-C₈-alkyl, alkoxy-(C₁-C₈) , acyloxy-(C₁-C₈), O-phenyl, phenyl, fluorine, chlorine, bromine, iodine, OH, NO₂, OSO₂CF₃, CN, COOH, CHO, SO₃H, SO₂R, SOR, NH₂, NH-alkyl-(C₁-C₈), N-alkyl₂-(C₁-C₈), CHal₃, NHCO-alkyl- (C₁-C₄) , N-alkyl-(C₁-C₄) -CO-alkyl-(C₁-C₄), COO-alkyl-(C₁-C₈), CONH₂, CO-alkyl-(C₁-C₈), NHCOH, NCOO-alkyl-(C₁-C₄), CO-phenyl, COO-phenyl, CHCH-CO₂-alkyl-(C₁-C₈), CHCHCO₂H, PO-phenyl₂, PO-alkyl₂-(C₁-C₄), where one of the radicals R^{1a} to R^{5a} can also be 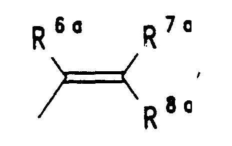 R^{6a} is hydrogen, alkyl(C₁-C₈), phenyl, O-alkyl-(C₁-C₈), fluorine,
R^{7a} and R^{8a} are, independently of one another, hydrogen, CN, CO₂H, CO₂-alkyl-(C₁-C₈), CONH₂, CONH-alkyl-(C₁-C₄), CON(alkyl)₂-(C₁-C₄), fluorine, CO₂-phenyl, alkyl, (C₁-C₈)-phenyl, PO(phenyl), PO(alkyl-(C₁-C₄))₂, CO-phenyl, CO-alkyl-(C₁-C₄), O-alkyl-(C₁-C₄), NH-alkyl-(C₁-C₄), PO₃H, SO₃H, SO₃-alkyl-(C₁-C₄), SO₂-alkyl-(C₁-C₄), O-phenyl,
by reaction of haloaromatics of the formula (II) 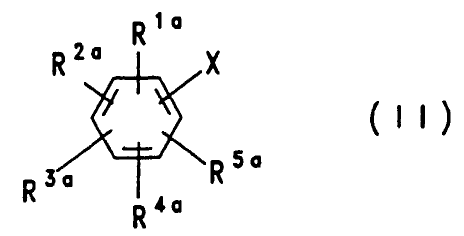 with olefins of the formula (III) 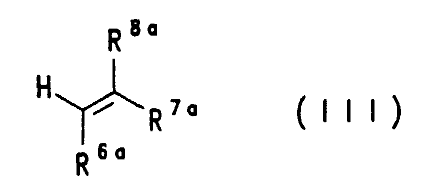 where
R^{1a} to R^{8a} are as defined above, where one of the radicals R^{1a} to R^{5a} can also be X and X is iodine, bromine, chlorine, OSO₂CF₃, OSO₂-phenyl, OSO₂CH₃, wherein a palladium compound of the formula (IV) 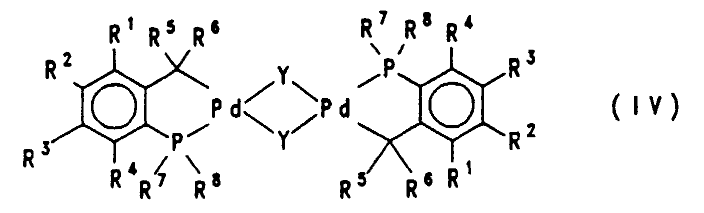 where
R¹, R², R³, R⁴, R⁵, R⁶ are, independently of one another, hydrogen, (C₁-C₄)-alkyl, (C₅-C₈)-cycloalkyl, (C₁-C₄)-alkoxy, fluorine, NH₂, NH-alkyl(C₁-C₄), N(alkyl)₂-(C₁-C₄), CO₂alkyl-(C₁-C₄), OCO-alkyl-(C₁-C₄) or phenyl,
or R¹ and R², R² and R³, R³ and R⁴, R⁵ and R⁶ together form an aliphatic or aromatic ring, and
R⁷, R⁸ are (C₁-C₈)-alkyl, (C₃-C₁₂)-cycloalkyl, substituted or unsubstituted aryl and
Y is an anion of an inorganic or organic acid, is used as catalyst with a ratio Pd:P = 1:1.

2. The process as claimed in claim 1, wherein, in formula (IV), R¹ to R⁶ are, independently of one another, hydrogen, (C₁-C₄)-alkyl, (C₅-C₈) -cycloalkyl, R⁷, R⁸ are phenyl, tolyl, xylyl, mesityl, alkyl-(C₁-C₈), cycloalkyl-(C₅-C₈) and
Y is acetate, propionate, benzoate, chloride, bromide, iodide, fluoride, sulfate, hydrogensulfate, nitrate, phosphate, tetrafluoroborate, tosylate, mesylate, acetylacetonate, hexafluoroacetylacetonate or pyrazolyl.

3. The process as claimed in claim 1, wherein the catalyst used is one of the compounds
trans-di-µ-acetato-bis[o-(di-o-tolylphosphino)benzyl]dipalladium(II)
trans-di-µ-chloro-bis[o-(di-o-tolylphosphino)benzyl]dipalladium(II)
trans-di-µ-bromo-bis [o-(di-o-tolylphosphino)benzyl]dipalladium(II)
trans-di-µ-iodo-bis[o-(di-o-tolylphosphino)benzyl]dipalladium(II)
trans-di-µ-acetato-bis[o-(dimesitylphosphino)-3,5-dimethylbenzyl]dipalladium(II)
trans-di-µ-chloro-bis[o-(dimesitylphosphino)-3,5-dimethylbenzyl]dipalladium(II)
trans-di-µ-bromo-bis[o-(dimesitylphosphino)-3,5-dimethylbenzyl]dipalladium(II)
trans-di-µ-iodo-bis-[o-(dimesitylphosphino)-3,5-dimethylbenzyl]dipalladium(II)
trans-di-µ-acetato-bis[o-(t-butyl-o-tolylphosphino)benzyl]dipalladium(II)
trans-di-µ-acetato-bis[o-(di-t-butylphosphino)benzyl]dipalladium(II)
trans-di-µ-acetato-bis[o-(cyclohexyl-o-tolylphosphino)benzyl]dipalladium(II).

4. The process as claimed in at least one of claims 1 to 3, wherein the catalyst is prepared in situ.

5. The process as claimed in at least one of claims 1 to 4, wherein, in formula (I):
R^{1a} to R^{5a} are, independently of one another, hydrogen, (C₁-C₈)-alkyl, (C₁-C₈)-alkoxy, (C₁-C₈)-acyloxy, phenyl, fluorine, chlorine, NO₂, CN, COOH, CHO, SO₂R, NH-(C₁-C₈)-alkyl, N[(C₁-C₈)alkyl]₂, COO-(C₁-C₈)-alkyl, CONH₂, CO-(C₁-C₈)-alkyl, CO-phenyl, COO-phenyl, PO-(phenyl)₂,
R^{6a} is hydrogen, (C₁-C₈)-alkyl,
R^{7a}, R^{8a} are, independently of one another, hydrogen, CN, CO₂H, CO₂-(C₁-C₈)-alkyl, CO₂-phenyl, (C₁-C₈)-alkyl, CO-phenyl, CO-(C₁-C₄)-alkyl.

6. The process as claimed in at least one of claims 1 to 4, wherein, in formula (I):
R^{1a} to R^{5a} are, independently of one another, hydrogen, (C₁-C₈)-alkyl, (C₁-C₈)-alkoxy, phenyl, fluorine, chlorine, NO₂, CN, COOH, CHO, COO-(C₁-C₈)-alkyl, CONH₂, CO-(C₁-C₈)-alkyl, CO-phenyl, PO-(phenyl)₂, R^{6a} is hydrogen,
R^{7a}, R^{8a} are, independently of one another, CN, CO₂H, CO₂-(C₁-C₈)-alkyl, CO₂-phenyl, CO-phenyl, CO-(C₁-C₄)alkyl.

7. The process as claimed in at least one of claims 1 to 6, wherein solvents used are dipolar aprotic solvents, in particular dialkyl sulfoxides, N,N-dialkylamides, alkylated lactams, preferably dimethyl sulfoxide, dimethylacetamide, dimethylformamide or N-methylpyrrolidone.

8. The process as claimed in at least one of claims 1 to 7, wherein the reaction is carried out at temperatures of from 20 to 200°C, in particular from 60 to 180°C, preferably from 100 to 150°C.

9. The process as claimed in at least one of claims 1 to 8, wherein the acid HX formed in the reaction is neutralized by adding a base, in particular an amine or an alkali metal or alkaline earth metal salt of a weak acid.

10. The process as claimed in claim 9, wherein the base used is an alkylamine or a carbonate, hydrogencarbonate or acetate of lithium, sodium, potassium, calcium or magnesium.

11. The process as claimed in at least one of claims 1 to 10, wherein salts of halides and pseudohalides of the alkali metals, alkaline earth metals and metals of transition groups VI to VIII are added.

12. The process as claimed in at least one of claims 1 to 10, wherein trialkylammonium or tetraalkylammonium, trialkylphosphonium or tetraalkylphosphonium or trialkylarsonium or tetraalkylarsonium salts are added.

## Revendications

1. Procédé pour la préparation d'oléfines aromatiques monofonctionnelles, bi- et polyfonctionnelles, de formule (I) 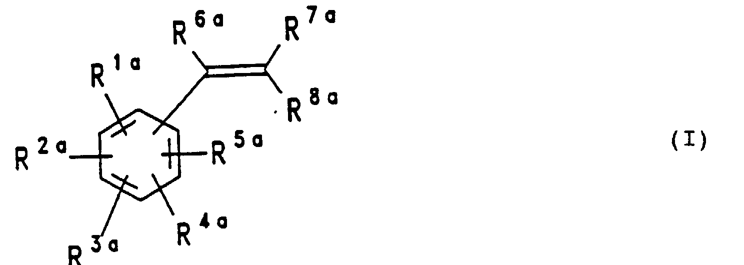 dans laquelle
R^{1a} à R^{5a}, indépendamment les uns des autres, représentent hydrogène, alkyle en C₁-C₈, alcoxy en C₁-C₈, acyloxy en C₁-C₈, O-phényle, phényle, fluor, chlore, brome, iode, OH, NO₂, OSO₂CF₃, CN, COOH, CHO, SO₃H, SO₂R, SOR, NH₂, NHalkyle en C₁-C₈, N-(alkyle en C₁-C₈)₂, CHal₃, NHCO-alkyle en C₁-C₄, N-alkyl en C₁-C₄-CO-alkyle en C₁-C₄, COO-alkyle en C₁-C₈, CONH₂, CO-alkyle en C₁-C₈, NHCOH, NCOO-alkyle en C₁-C₄, CO-phényle, COO-phényle, CHCHCO₂-alkyle en C₁-C₈, CHCHCO₂H, PO-phényle₂, PO(alkyle en C₁-C₄)₂, l'un des radicaux R^{1a} à R^{5a} pouvant également représenter 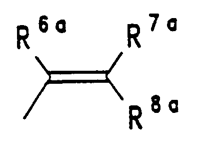
R^{6a} représente hydrogène, alkyle en C₁-C₈, phényle, O-alkyle en C₁-C₈, fluor,
R^{7a} et R^{8a} représentent, indépendamment l'un de l'autre, hydrogène, CN, CO₂H, CO₂-alkyle en C₁-C₈, CONH₂, CONH-alkyle en C₁-C₄, CON(alkyle en C₁-C₄)₂, fluor, CO₂-phényle, alkyl en C₁-C₈-phényle, PO-(phényle), PO-(alkyle en C₁-C₄)₂, CO-phényle, CO-alkyle en C₁-C₄, O-alkyle en C₁-C₄, NH-alkyle en C₁-C₄, PO₃H, SO₃H, SO₃-alkyle en C₁-C₄, SO₂-alkyle en C₁-C₄, O-phényle,
par réaction de composés aromatiques halogénés de formule générale (II) : 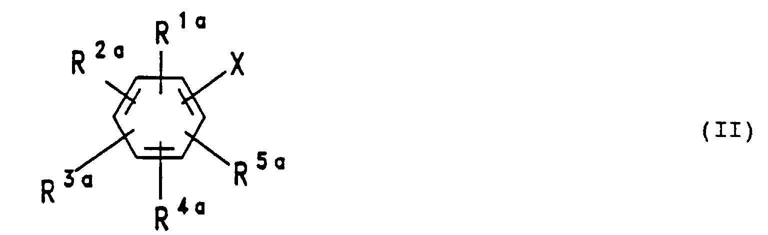 avec des oléfines de formule générale (III) : 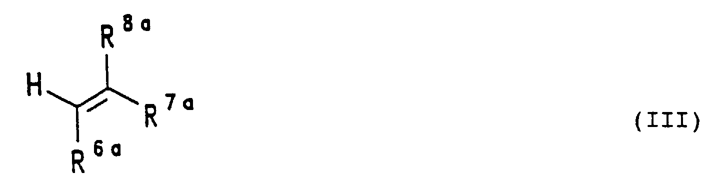 dans lesquelles R^{1a} à R^{8a} possèdent les significations indiquées, l'un des radicaux R^{1a} à R^{5a} pouvant également remplacer X et X représente l'iode, le brome, le chlore, OSO₂CF₃, OSO₂phényle, OSO₂CH₃, caractérisé en ce que l'on utilise comme catalyseur un composé du palladium de formule générale (IV) : 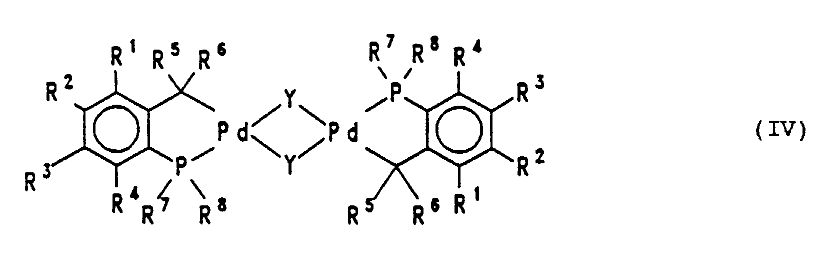 dans laquelle
R¹, R², R³, R⁴, R⁵, R⁶, indépendamment les uns des autres, représentent hydrogène, alkyle en C₁-C₄, cycloalkyle en C₅-C₈, alcoxy en C₁-C₄, fluor, NH₂, NH-alkyle en C₁-C₄, N-(alkyle en C₁-C₄)₂, CO₂-(alkyle en C₁-C₄)₂, OCO-alkyle en C₁-C₄, ou phényle,
ou R¹ et R², R² et R³, R³ et R⁴, R⁵ et R⁶ forment ensemble un cycle aliphatique ou aromatique, et
R⁷, R⁸ sont alkyle en C₁-C₈, cycloalkyle en C₃-C₁₂, aryle substitué ou non substitué,
Y représente un anion d'un acide inorganique ou organique,
dans une proportion Pd:P = 1:1.

2. Procédé selon la revendication 1, caractérisé en ce que dans la formule (IV) R¹ à R⁶ représentent, indépendamment les uns des autres, hydrogène, alkyle en C₁-C₄, cycloalkyle en C₅-C₈,
R⁷, R⁸ représentent phényle, tolyle, xylyle, mésityle, alkyle en C₁-C₈, cycloalkyle en C₅-C₈, et
Y représente acétate, propionate, benzoate, chlorure, bromure, iodure, fluorure, sulfate, sulfate acide, nitrate, phosphate, tétrafluoroborate, tosylate, mésylate, acétylacétonate, hexafluoroacétylacétonate, ou pyrazolyle.

3. Procédé selon la revendication 1, caractérisé en ce que l'on utilise comme catalyseurs les composés :
trans-di-µ-acétato-bis[o-(di-o-tolylphosphino)-benzyl]-dipalladium(II) ;
trans-di-µ-chloro-bis[o-(di-o-tolylphosphino)-benzyl]-dipalladium(II) ;
trans-di-µ-bromo-bis[o-(di-o-tolylphosphino)-benzyl]-dipalladium(II) ;
trans-di-µ-iodo-bis[o-(di-o-tolylphosphino)-benzyl]-dipalladium(II) ;
trans-di-µ-acétato-bis[o-(dimésitylphosphino)-3,5-diméthylbenzyl]-dipalladium(II) ;
trans-di-µ-chloro-bis[o-(dimésitylphosphino)-3,5-diméthylbenzyl]-dipalladium(II) ;
trans-di-µ-bromo-bis[o-(dimésitylphosphino)-3,5-diméthylbenzyl]-dipalladium(II) ;
trans-di-µ-iodo-bis[o-(dimésitylphosphino)-3,5-diméthylbenzyl]-dipalladium(II) ;
trans-di-µ-acétato-bis[o-(tertio-butyl-o-tolylphosphino)-benzyl]-dipalladium(II) ;
trans-di-µ-acétato-bis[o-(di-tertio-butylphosphino)-benzyl]-dipalladium(II) ;
trans-di-µ-acétato-bis[o-(cyclohexyl-o-tolylphosphino)-benzyl]-dipalladium(II).

4. Procédé selon au moins l'une des revendications 1 à 3, caractérisé en ce que le catalyseur est préparé *in situ*.

5. Procédé selon au moins l'une des revendications 1 à 4, caractérisé en ce que, dans la formule (I)
R^{1a} à R^{5a}, indépendamment les uns des autres, représentent hydrogène, alkyle en C₁-C₈, alcoxy en C₁-C₈, acyloxy en C₁-C₈, phényle, fluor, chlore, NO₂, CN, COOH, CHO, SO₂R, NH-alkyle en C₁-C₈, N-(alkyle en C₁-C₈)₂, COO-alkyle en C₁-C₈, CONH₂, CO-alkyle en C₁-C₈, CO-phényle, COO-phényle, PO-(phényle)₂,
R^{6a} représente hydrogène, alkyle en C₁-C₈,
R^{7a}, R^{8a} représentent, indépendamment l'un de l'autre, hydrogène, CN, CO₂H, CO₂-alkyle en C₁-C₈, CO₂-phényle, alkyle en C₁-C₈, CO-phényle, CO-alkyle en C₁-C₄.

6. Procédé selon au moins l'une des revendications 1 à 4, caractérisé en ce que, dans la formule (I) :
R^{1a} à R^{5a}, indépendamment les uns des autres, représentent hydrogène, alkyle en C₁-C₈, alcoxy en C₁-C₈, phényle, fluor, chlore, NO₂, CN, COOH, CHO, COO-alkyle en C₁-C₈, CONH₂, CO-alkyle en C₁-C₈, CO-phényle, PO-(phényle)₂,
R^{6a} représente hydrogène,
R^{7a}, R^{8a}, indépendamment l'un de l'autre, représentent CN, CO₂H, CO₂-alkyle en C₁-C₈, CO₂-phényle, CO-phényle, CO-alkyle en C₁-C₄.

7. Procédé selon au moins l'une des revendications 1 à 6, caractérisé en ce que l'on utilise comme solvants des solvants aprotiques dipolaires, en particulier des dialkylsulfoxydes, des N,N-dialkylamides, des lactames alkylés, de préférence le diméthylsulfoxyde, le diméthylacétamide, le diméthylformamide ou la N-méthylpyrrolidone.

8. Procédé selon au moins l'une des revendications 1 à 7, caractérisé en ce que la réaction est effectuée à des températures de 20 à 200°C, en particulier de 60 à 180°C, de préférence de 100 à 150°C.

9. Procédé selon au moins l'une des revendications 1 à 8, caractérisé en ce que l'acide HX qui se forme lors de la réaction est capturé par addition d'une base, en particulier d'une amine ou d'un sel de métal alcalin ou alcalino-terreux d'un acide faible.

10. Procédé selon la revendication 9, caractérisé en ce qu'en tant que bases, on utilise des alkylamines ou les carbonates, carbonates acides ou acétates de lithium, sodium, potassium, calcium ou magnésium.

11. Procédé selon au moins l'une des revendications 1 à 10, caractérisé en ce qu'on ajoute des sels d'halogénures ou de pseudo-halogénures des métaux alcalins, alcalino-terreux et métaux des sous-groupes VI à VIII.

12. Procédé selon au moins l'une des revendications 1 à 10, caractérisé en ce que l'on ajoute des sels de tri- ou tétra-alkylammonium, -phosphonium ou -arsonium.
